# EUROPEAN PATENT APPLICATION

(11) **EP 4 327 725 A1**
(43) Date of publication of application: **28.02.2024**
(21) Application number: 22791941.2
(22) Date of filing: 11.04.2022
(51) Int. Cl.: A61B 5/00, A61B 5/145, G16H 50/50, G16H 50/30, G01N 24/08, G01N 33/487, G01N 30/72

(54) **METHOD FOR EARLY PREDICTION OF PRETERM BIRTH, USING RETINOID METABOLITES**

(30) Priority: 23.04.2021 KR 20210053232
(71) Applicant: Ewha University-Industry Collaboration Foundation, Seoul 03760 (KR)
(72) Inventor: KIM, Young Ju, Seoul 07980 (KR); YOU, Young-Ah, Seoul 05085 (KR); ANSARI, Abuzar, Goyang-si, Gyeonggi-do 05085 (KR); KIM, Soo-Min, Incheon 21058 (KR); PARK, Sunwha, Seoul 07997 (KR); LEE, Ga-In, Cheonan-si, Chungcheongnam-do 31233 (KR)
(74) Representative: Gevers Patents
(86) International application number: PCT/KR2022/005190
(87) International publication number: WO 2022/225241

(57) **Abstract**

The present invention relates to a method for early prediction of preterm birth, using retinoid metabolites.

## Description

### [TECHNICAL FIELD]

The present invention relates to a method for early prediction of preterm birth using retinoid metabolites.

### [BACKGROUND ART]

Preterm birth (PTB) refers to giving birth at less than 37 weeks, and according to recent statistics of 107 countries in the Asian region, it occurs with a frequency of about 10.6%. In case of Korea, the preterm birth rate was shown as about 4.8% in 2006, but it almost nearly doubled to 7.2% in 2016, and the preterm birth rate shows a trend of increasing. Preterm birth has a risk of increasing mortality in newborns and children under 5 years of age, and has an increased probability of causing problems such as neurodevelopment and the like, in the short term, and has a risk of increasing prevalence of type 2 diabetes, obesity, high blood pressure and the like.

The pathogenesis of preterm birth is not yet clearly discovered, and risk factors commonly associated with spontaneous labor include reproductive system infections, multiple pregnancies, second and third trimester bleeding, an anamnesis of previous preterm birth and the like. In addition, approximately 75% of preterm birth occurs with premature labor and rupture of membrane, and cervical incompetency and chorioamnionitis associated thereto.

Korea is a country with a low delivery rate, but the preterm birth rate of less than 37 weeks is steadily increasing, so it threatens the health of mothers and newborns and further causes economic loss costs. Until now, in order to overcome preterm birth, preterm birth has been predicted through fatal fibronectin, CRP measurement, cervical length measurement, and the like, but the sensitivity of the preterm birth prediction rate is still low.
Therefore, when signs of preterm birth appear at an early week, increasing maturity of a newborn by delaying delivery weeks through appropriate treatment is a critical key point to the health and quality of life of the mother and newborn and cost.

### [DETAILED DESCRIPTION OF THE INVENTION]

### [TECHNICAL PROBLEM]

One embodiment of the present invention is to provide a method for predicting preterm birth or a method for providing information for predicting preterm birth, which can predict preterm birth using the level of retinoid metabolites in a biological sample.

Another embodiment of the present invention relates to a kit for predicting preterm birth, which can predict preterm birth using the level of retinoid metabolites in a biological sample.

Other embodiment of the present invention relates to a system for predicting preterm birth, which can predict preterm birth using the level of retinoid metabolites in a biological sample.

### [TECHNICAL SOLUTION]

One embodiment of the present invention relates to a method for predicting preterm birth or a method for providing information for predicting preterm birth, comprising quantifying retinoid metabolites in a biological sample.

Another embodiment of the present invention relates to a composition for predicting preterm birth, comprising an agent capable of quantifying retinoid metabolites in a biological sample.

Other embodiment of the present invention relates to a kit for predicting preterm birth, comprising an agent for quantification, or a device for quantification, which quantifies retinoid metabolites in a biological sample.

Other embodiment of the present invention relates to a system for predicting preterm birth, comprising a quantification unit which quantifies retinoid metabolites in a biological sample.

Hereinafter, the present invention will be described in more detail.

One embodiment of the present invention relates to a method for providing information for predicting preterm birth, a method for predicting preterm birth, a method for providing information for diagnosing preterm birth, or a method for diagnosing preterm birth, comprising quantifying retinoid metabolites in a biological sample.

In the present description, the terms, "preterm birth", "premature birth", "preterm birth", "PTB", and the like have the same meaning, and mean birth of a fetus at less than 37 weeks.

According to the example of the present application, the markers for retinoid metabolites for preterm birth according to the present invention, are analyzed using a blood sample of a mother, and showed a significant difference between the preterm birth group and term birth group, and therefore, preterm birth can be predicted using the markers for retinoid metabolites according to one embodiment of the present invention.

In the present invention, the term of "diagnosis of a risk of preterm birth" or "prediction of a risk of preterm birth" refers to determining whether a pregnant woman is likely to have preterm birth, whether she is likely to have the relatively high possibility of preterm birth, or whether she is likely to show signs of preterm birth.

The present invention may be used to delay or prevent preterm birth through special and suitable management for pregnant women with a high risk of preterm birth. In addition, the present invention may be clinically used to determine treatment by selecting the most appropriate treatment method by diagnosing preterm birth at an early stage.

In the present description, the term "or" is used as a meaning including "and/or", unless the content is clearly specified.

In the present description, the term, "marker", "marker for diagnosis", "marker for diagnosing", "diagnosis marker" or "probe" is a substance that becomes a standard for predicting a preterm birth pregnant woman and distinguishing her from a normal birth group, and means bioorganic molecules that show a significant difference in a sample of a pregnant woman predicted to have preterm birth. According to one embodiment of the present invention, the marker and the like may comprise metabolites in a biological sample.

The metabolite is a substance produced as a result of metabolism in vivo, and may be expressed as a metabolite, an intermediate metabolite, a metabolic intermediate or the like, and refers to entire metabolome of small molecules that mainly occur during a cellular process. For example, according to one embodiment of the present invention, the metabolite may be a retinoid metabolite. Specifically, the metabolite may comprise at least one selected from the group consisting of all trans-retinal, 13-cis-retinoic acid and retinyl palmitate. As one example, the metabolite may comprise at least one selected from the group consisting of all trans-retinal and 13-cis-retinoic acid.

In the present description, the term "biological sample" means a sample isolated or collected from a test subject, or a test individual, for example, a pregnant woman. The biological sample may be a sample derived from a pregnant woman, specifically, a blood sample of a pregnant woman, for example, at least one selected from the group consisting of whole blood, plasma and serum.

The method for providing information for predicting preterm birth, method for predicting preterm birth, method for providing information for diagnosing preterm birth, or method for diagnosing preterm birth according to one embodiment of the present invention may further comprise comparing the level of the quantified metabolite with a control group.

For example, it may further comprise predicting the pregnant woman from which the biological sample is isolated has a risk of preterm birth, when the metabolite is at least one selected from the group consisting of all trans-retinal, 13-cis-retinoic acid and retinyl palmitate, and the level of the quantified metabolite is higher than a control group. The control group may be a mother normally giving birth without preterm birth, a mother giving birth at 37 weeks or more of pregnancy, or a term birth mother.

In the present description, the term "increase" means an increase in quantity, an increase in level, an increase in distribution, an increase of distribution rate, or an increase in concentration, or the like, from a comparison control group, for example, an established control group or standard control group (for example, an average level of a marker metabolite shown in a biological sample of a mother normally giving birth, or a biological sample of a mother giving birth at 37 weeks or more of pregnancy. Specifically, the distribution rate may be a relative content, or a relative concentration of a specific metabolite in a biological sample as a result of analysis of liquid chromatography-mass spectrometer and the like.

For example, the increase may be an increase of more than 1 time, 1.1 times or more, 1.15 times or more, 1.2 times or more, 1.25 times or more, 1.3 times or more, 1.35 times or more, 1.4 times or more, 1.45 times or more, or 1.5 times or more of a control group value, or a statistically significant increase, but those skilled in the art will be able to clearly understand the significantly increased change compared to the control group, even if the absolute increased amount is not specified. The control group value may be an average value of control group values of for example, at least 2 or more, 3 or more, 4 or more, 5 or more, 10 or more, 15 or more, 20 or more, 25 or more, 30 or more, or 35 or more.

For example, when the concentration of all trans-retinal in a biological sample is 1.5 times or more of a control group value, it may be predicted that a pregnant woman from which the biological sample is isolated has a risk of preterm birth.

For example, when the concentration of 13-cis-retinoic acid in a biological sample is 1.4 times or more of a control group value, it may be predicted that a pregnant woman from which the biological sample is isolated has a risk of preterm birth.

For example, when the concentration of retinyl palmitate in a biological sample is 1.5 times or more of a control group value, it may be predicted that a pregnant woman from which the biological sample is isolated has a risk of preterm birth.

For example, when the concentration of all trans-retinal in a biological sample is 2.48 ppb or more, it may be predicted that a pregnant woman from which the biological sample is isolated has a risk of preterm birth.

For example, when the concentration of 13-cis-retinoic acid in a biological sample is 5.56 ppb or more, it may be predicted that a pregnant woman from which the biological sample is isolated has a risk of preterm birth.

For example, when the concentration of retinyl palmitate in a biological sample is 100.33 ppb or more, it may be predicted that a pregnant woman from which the biological sample is isolated has a risk of preterm birth.

The terms showing the increase, for example, the terms of "high", "more", "higher", "significant high", "statistically significant high" and the like are used as the same meaning as described above. In contrast, the terms of "not significantly", "equally", "substantially same", "substantially unchanged" and the like mean that there is little change or no significant change from a standard control group value, such as within ±10%, within ±5%, within ±3%, or within ±2% or the like of the standard control group.

In the present description, the term "control group" is a subject for comparing the level of the metabolite according to the present invention to predict preterm birth, and may be interchangeably used with a term such as a comparison control group, a standard control group, or a normal control group. For example, the control group may be a mother not having preterm birth, a mother normally giving birth, or a mother giving birth at 37 weeks or more of pregnancy.

The quantifying the metabolites may be performed by at least one selected from the group consisting of liquid chromatography-mass spectrometer (LC/MS), nuclear magnetic resonance spectrometer, ultraviolet spectrometer, infrared spectrometer, fluorescence spectrometer, ELISA (enzyme-linked immunosorbent assay), and mass spectrometer.

The preterm birth according to one embodiment of the present invention may be not preterm birth by liver dysfunction. According to the example of the present application, the preterm birth group showing a significant difference of the retinoid metabolite preterm birth marker according to the present invention and the normal control group, did not show a significant difference in the liver function-related index, specifically, the hepatic cell damage factor level such as alanine aminotransferase (ALT) and aspartate aminotransferase (AST) and the like, and more specifically, did not show a significant difference in the hepatic cell damage factor level at the third stage of pregnancy. Accordingly, the preterm birth according to one embodiment of the present invention may be not preterm birth by liver dysfunction.

In other words, the subject mother for predicting preterm birth according to one embodiment of the present invention, may be a mother having no liver dysfunction. Specifically, the mother may be a mother having no liver dysfunction at the third stage of pregnancy.

For example, the mother may be a mother having no significant difference in the liver function-related index, or hepatic cell damage factor level from a normal control group. Specifically, the mother may be a mother having no significant difference in the liver function-related index, or hepatic cell damage factor level from a normal control group at the third stage of pregnancy.

For example, the mother may be a mother having no significant difference in the level of alanine aminotransferase (ALT) and/or aspartate aminotransferase (AST) from a normal control group.

For example, the mother may be a mother with AST of 50 U/L or less, 45 U/L or less, 40 U/L or less, 35 U/L or less, 30 U/L or less, 25 U/L or less, or 20 U/L or less.

For example, the mother may be a mother with ALT of 50 U/L or less, 45 U/L or less, 40 U/L or less, 35 U/L or less, 30 U/L or less, 25 U/L or less, 20 U/L or less, or 15 U/L or less.

For example, the mother may be a mother with an AST/ALT ratio of 0.1 to 2, 0.1 to 1.8, 0.1 to 1.5, 0.3 to 2, 0.3 to 1.8, 0.3 to 1.5, 0.5 to 2, 0.5 to 1.8, 0.5 to 1.5, 0.7 to 2, 0.7 to 1.8, 0.7 to 1.5, 1 to 2, 1 to 1.8, or 1 to 1.5.

Other embodiment of the present invention relates to a composition for predicting preterm birth, comprising an agent capable of quantifying retinoid metabolites in a biological sample.

The retinoid metabolites are as described above, and for example, may comprise at least one selected from the group consisting of all trans-retinal, 13-cis-retinoic acid and retinyl palmitate. As one example, the metabolites may comprise at least one selected from the group consisting of all trans-retinal and 13-cis-retinoic acid.

The agent may quantify the quantity, level, distribution, distribution rate, or concentration of metabolites in a biological sample.

For example, the agent may quantify an increase in at least one selected from the group consisting of all trans-retinal, 13-cis-retinoic acid and retinyl palmitate, as one example, at least one selected from the group consisting of all trans-retinal and 13-cis-retinoic acid, for example, an increase in quantity, level, distribution, distribution rate, or concentration.

The metabolites may be quantified by at least one selected from the group consisting of liquid chromatography-mass spectrometer (LC/MS), nuclear magnetic resonance spectrometer, ultraviolet spectrometer, infrared spectrometer, fluorescence spectrometer, ELISA (enzyme-linked immunosorbent assay), and mass spectrometer, but not limited thereto.

Other embodiment of the present invention relates to a kit for predicting preterm birth, comprising an agent for quantification, or a device for quantification which quantifies retinoid metabolites in a biological sample. The retinoid metabolites are as described above.

The kit may quantify an increase in at least one selected from the group consisting of all trans-retinal, 13-cis-retinoic acid and retinyl palmitate, as one example, at least one selected from the group consisting of all trans-retinal and 13-cis-retinoic acid, for example, an increase in quantity, level, distribution, distribution rate, or concentration.

The quantification device may be at least one selected from the group consisting of liquid chromatography-mass spectrometer (LC/MS), nuclear magnetic resonance spectrometer, ultraviolet spectrometer, infrared spectrometer, fluorescence spectrometer, ELISA (enzyme-linked immunosorbent assay), and mass spectrometer, but not limited thereto.

Other embodiment of the present invention relates to a device, equipment, or system for predicting or diagnosing preterm birth. Specifically, it relates to a device, equipment, or system capable of performing all or a part of the steps of the method for predicting preterm birth, method for providing information for predicting preterm birth, method for diagnosing preterm birth, or method for providing information for diagnosing preterm birth described above. The device, equipment, or system may perform all or a part of the steps using the composition for predicting preterm birth or kit for predicting preterm birth or the like according to one embodiment of the present invention.

For example, the device, equipment, or system may obtain a biological sample of a test subject, for example, a blood sample isolated from a test subject pregnant woman, quantify the quantity or concentration of the marker metabolites according to the present invention in the sample, compare the quantity or concentration with a control group, for example, a control group mother normally giving birth, and display a result indicating whether there is a risk of preterm birth of the test subject. Accordingly, the device, equipment or system according to one embodiment of the present invention may comprise a quantification unit which quantifies retinoid metabolites in a biological sample.

Specifically, the device, equipment, or system may comprise an input unit which receives input of a biological sample of a test subject; a quantification unit which quantifies the marker metabolites according to the present invention in the sample; a calculation unit which compares the level of the quantified metabolites with a numerical value of a control group; and a display unit which displays a result indicating whether there is a risk of preterm birth of the test subject according to the comparison result

The marker metabolites are as described above, and for example, may include at least one selected from the group consisting of all trans-retinal, 13-cis-retinoic acid and retinyl palmitate. As one example, the marker metabolites may comprise at least one selected from the group consisting of all trans-retinal, 13-cis-retinoic acid and retinyl palmitate

When it is calculated that the level of at least one selected from the group consisting of all trans-retinal, 13-cis-retinoic acid and retinyl palmitate is higher than the control group in the calculation unit, the display unit may display that the test subject has a risk of preterm birth.

The biological sample may be isolated from a pregnant woman. The biological sample may be a blood sample, and as one example, it may be at least one selected from the group consisting of whole blood, plasma and serum.

The quantification unit may quantify metabolites with at least one selected from the group consisting of liquid chromatography-mass spectrometer (LC/MS), nuclear magnetic resonance spectrometer, ultraviolet spectrometer, infrared spectrometer, fluorescence spectrometer, ELISA (enzyme-linked immunosorbent assay), and mass spectrometer.

### [ADVANTAGEOUS EFFECTS]

By using the retinoid metabolite preterm birth marker according to one embodiment of the present invention, preterm birth can be predicted at an early stage, and non-invasive and highly accurate prediction of preterm birth is possible.

### [BRIEF DESCRIPTION OF THE DRAWINGS]

FIG. 1 is a flowchart which shows the process of selecting a test subject according to one embodiment of the present invention.
FIG. 2 shows categories clearly divided for PTB compared to TB in RPC positivity (Q2 = 0.072; R2 = 0.085).
FIG. 3 shows a scatter plot of pathway impact analysis, and the y-axis indicates a p-value.
FIG. 4 is diagrams which show retinoid metabolites exhibiting a change in a plasma sample of a preterm birth mother through retinoid metabolite analysis (39TB, 20PTB).
FIG. 5 is diagrams which show ROC curve analysis for metabolites, and shows the prediction performance of preterm birth biomarkers using ROC curves of sensitivity and specificity.

### [MODE FOR INVENTION]

Hereinafter, the present invention will be described in more detail by the following examples. However, these examples are intended to illustrate the present invention only, but the scope of the present invention is not limited by these examples.

### Experimental materials and methods

### Example 1. Research subjects

Pregnant women who visited Ewha Womans University Medical Center for prenatal tests and delivery were recruited. The subjects were divided into a discovery group for analyzing metabolite profiles (10 TB and 11 PTB), and a validation group for analyzing retinoid metabolites (39 TB and 20 PTB). The research subjects were asymptomatic singleton pregnant women with asymptomatry, or hospitalized pregnant women with preterm labor (PTL) and/or premature rupture of membrane (pPROM) between 16 weeks and 35 weeks and 5 days of pregnancy.

Preterm labor (PTL) was diagnosed in patients with regular uterine contractions and 4 times or more contractions within 20 minutes, or 8 times or more contractions within 60 minutes, by detecting by electrocardiography. In order to diagnose premature rupture of membrane (pPROM), a sterile speculum examination to detect fixation of amniotic fluid in the vaginal cavity and a nitrazine test to detect membrane rupture were conducted. The gestational age was determined using the first day of last menstruation and ultrasonography. When the pregnant women visited for prenatal tests or were hospitalized for giving birth, blood samples were collected and stored at -80 °C until metabolite analysis was performed.

The present study was approved by the Ewha Womans University Hospital Institutional Review Board (EUMC) (EUMC 2018-07-007-010). All experiments were performed according to approved guidelines, and informed consent was obtained from all subjects.

### Example 2. Preparation of mother blood samples

A plasma sample of 200 µL was precipitated with a solvent mixture (acetonitrile/methanol/acetone; 1:1:1, v/v) of 600 µL, and then mixed by vortex for 3 minutes and scored at -20°C for 30 minutes. The precipitated sample was centrifuged at 10,000g, 4°C for 10 minutes, and then supernatant of 600µl was transferred to another test tube and evaporated and dried under a gentle nitrogen stream. The residue was reconstituted with 50% methanol of 200µl and then a sample of 5µl (RPC separation mode) was analyzed by UPLC-LTQ-Orbitrap MS.

### Example 3. Metabolite profiling by LTQ-Orbitrap MS

Metabolite profiling was performed using Ultimate 3000 UHPLC 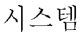 (Thermo Fisher Scientific, San Jose, CA, USA) combined with LTQ-OrbitrapVelos Pro hybrid mass spectrometer (Thermo Fisher Scientific) equipped with an electrospray source operating in a positive mode (ESI+). Reversed-phase separation was performed in Acquity ^{™} UPLC BEH C18 column (2.1mm × 100mm, 1.7µm, Waters, Milford, MA, USA) UPLC analysis column. As the mobile phase solvents, 95% water, 5% ACN and 0.1% formic acid (mobile phase A) and 95% ACN, 5% water and 0.1% formic acid (mobile phase B) were used. The elution gradient was as follows: to 0-3 minutes, 100% mobile phase A; to 3~10 minutes, linear increase to 50% mobile phase; to 10-12 minutes, linear increase of mobile phase B to 50-90%; to 12-15 minutes, maintaining a linear increase of 100% mobile phase A; to 15-18 minutes, re-equilibration to 100% mobile phase A. The column was maintained at 50 °C; and the total run time was 18 minutes. For analysis, a 5µl aliquot of each sample was injected. The samples were maintained at 4 °C in an auto sampler during analysis.

### Example 4. Preparation of standard stock solution for validation of retinoid metabolites

In order to analyze the metabolic pathways of TB and PTB, it was determined to analyze retinoid metabolites. For retinol (ROH), retinyl acetate (RAc), retinyl palmitate (RP), all-trans retinal (at-RAL), all-trans retinoic acid (at-RA), 13cis-retinoic acid (13cis-RA), retinyl acetate-D6 (RAc-D6), all-trans retinal-D6 (at-RAL-D6), and all-trans retinoic acid-D6 (at-RA-D6), storage solutions of all indicated analytical reference standards and internal standards of retinoids were uniformly dissolved in methanol so that the final concentration was to be 2000 ppm. Retinol-D6 (ROH-D6) was exceptionally prepared at a final concentration of 1000 ppm. The solutions were stored at a temperature of -20°C until use. The calibration curve for final quantification of target retinoids was prepared by serially diluting these standard stock solutions 4 times in a range of 800 ~ 0.05 ppb. ROH, RAc, at-RAL and at-RA standards were purchased from Cayman Chemicals, Inc. (Ann Arbor, Michigan), and RP, and 13cis-RA were purchased from Sigma Aldrich Corp. (St. Louis, USA, Missouri). All the 4 isotope-labeled internal standards mentioned above were purchased from Cambridge Isotopes Laboratories, Inc. (Tewksbury, USA, Massachusetts).

### Example 5. Sample preparation

In order to effectively extract target retinoids in plasma samples, a liquid-liquid extraction method was used. To each of the plasma samples of 200 µL, an isotope-labeled internal standard mixture was added, and before precipitating serum protein with acetonitrile of 200 µL, vortexing was carried out for a moment. After vortexing for 1 minute, methyl-tert-butyl ether of 1.2 mL was further added to each tube and vortexing was performed for 1 minute again. Then, the samples were centrifuged at 13,000 rpm, 4°C for 10 minutes, and the upper organic layer was finally transferred to a new test tube. The transferred supernatant was dried with nitrogen gas at a room temperature and the residue was reconstituted with methanol of 20µL. After vortexing for 5 minutes, the supernatant was finally transferred to a clean glass MS vial tube and a cap was closed for additional LC-MS/MS analysis.

### Example 6. LC-MS/MS analysis

Chromatographic separation of plasma sample extracts was performed on Accucore C 18 column (2.1x100mm, 2.6µm particle size, Thermo ScientificTM, USA), and the temperature was maintained at 30°C throughout the experiment. The injection volume of the samples was fixed at 2 µL. The target retinoids were separated within 22 minutes under a mobile phase composition consisting of 80% acetonitrile (A) and 100% methanol (B), and both of them were modified into 0.1% formic acid. The mobile phase system was initially maintained in an isocratic mode at 100% of phase A for 7 minutes, and the flow rate was linearly increased from 0.2 mL/min to 0.4 mL/min. After that, immediately, it was changed to 100% phase B and maintained up to 15 minutes and the flow rate was maintained at 0.4mL/min. For additional 2 minutes, the system gradually returned to 100% phase A using a linear gradient and the flow rate was reduced to 0.2mL/min again. To achieve equilibrium again, the conditions were maintained for 5 minutes. After that, column effluent was analyzed using Agilent 6460C Triple Quadrupole LC-MS/MS system equipped with an ESI (electrospray ionization) source. The mass spectrometer was operated in a cation mode using spray gas. The temperature of the heated sprayer was set to 300°C with an ion spray voltage of 4,500. Quantification of retinoids was performed in a multiple reaction monitoring (MRM) mode by selecting the precursor ion of [M+H-fatty acid-H2O]+ for retinol (ROH), retinol-D6 (ROH-D6), retinyl acetate (RAc), retinyl acetate-D6 (RAc-D6) and retinyl palmitate (RP), and selecting [M+H]+ for at-RAL, at-RAL-D6, at-RA, 9cis-RA, 13cis-RA and at-RA-D6. The optimized MRM transitions, MS parameters and LC retention times of all the mentioned analytes were summarized in Table 1. For final quantification, each peak area of the target retinoids was normalized to the response of the corresponding internal standard (0.5ppm ROH-D6 in case of ROH; 0.25ppm RAc-D6 in case of Rac and RP; 0.25ppm at-RAL-D6 in case of at-RAL; at-RA, 9cis-RA, 1ppm at-RA-D6 in case of 13cis-RA).

**[Table 1]**

| LC retention times, MRM transitions and MS parameters for target analytes | | | | | | |
|---|---|---|---|---|---|---|
| * Internal standards | | | | | | |
| **Analytes** | **Rₜ (min)** | **polarity** | **Precursor (m/z)** | **Product (m/z)** | **Fragmentor Energy (V)** | **Collision Energy (V)** |
| *13Cis*-retinoic acid | 3.128 | positive | 301.2 | 123.1 | 105 | 12 |
| *9Cis*-retinoic acid | 3.334 | positive | 301.2 | 123.1 | 105 | 12 |
| *All-trans* retinoic acid | 3.530 | positive | 301.1 | 159.1 | 115 | 18 |
| Retinoic acid-D6* | 3.531 | positive | 307.2 | 165.2 | 115 | 18 |
| *All-trans* retinal | 4.679 | positive | 285.2 | 161.1 | 115 | 2 |
| Retinal-D6* | 4.679 | positive | 291.2 | 69 | 95 | 22 |
| Retinol | 3.674 | positive | 269.2 | 93.1 | 115 | 18 |
| Retinol-D6* | 3.674 | positive | 275.2 | 96 | 110 | 14 |
| Retinyl acetate | 6.889 | positive | 269.2 | 96 | 108 | 21 |
| Retinyl acetate-D6* | 6.889 | positive | 275.2 | 96 | 115 | 16 |
| Retinyl palmitate | 13.982 | positive | 269.1 | 96 | 111 | 21 |

### Example 7. Statistical analysis

Basic characteristics of the research groups were compared using Student's t-test for continuous variables and chi square test for categorical variables. Multivariate analysis was performed in order to obtain a list of variables from the variation of metabolic profiles in the plasma of TB and PTB pregnant women. Multivariate analysis was performed using SIMCA-P software v14.0 of Umetrics (Umea, Sweden). The pathway impact analysis and heat map visualization were performed by Metaboanalyst 3.0 (Montreal, QC, Canada), a web-based metabolomics data processing tool and visualization metabolomics. The pathway mapping and chemical similarity analysis were generated by R version 3.2.2, MetaMapp and CytoScape 3.4.0 (Boston, MA, USA). In validation using LC-MS/MS, differential metabolite levels were analyzed using a generalized linear model adjusted for gestational age at sampling time. The diagnosis accuracy of PTB was summarized by applying the AUC of the ROC curve. Statistically, p<0.05 was considered significant. In the statistical analysis, the statistical package for social science (SPSS, version 2.0 Chicago, IL, USA) and on-line MEDCALC software were used.

### Experimental results

### 1. Clinical characteristics of experimental subjects

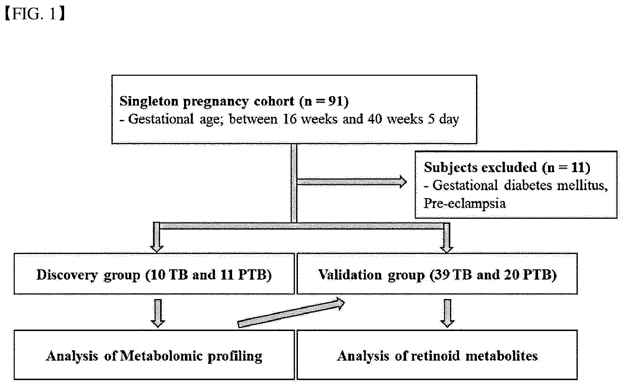

In order to confirm the metabolomics characteristics of preterm birth (PTB) (discovery group; TB, n = 10; PTB, n = 11; Table 2) and verify target metabolites (validation group; TB, n = 39; PTB n = 20; Table 3), peripheral blood was collected from pregnant women between 16 weeks and 40 weeks and 5 days of pregnancy (FIG. 1). FIG. 1 is a flow chart showing the process of selecting test subjects. The clinical characteristics of the preterm birth mothers and term mothers of the discovery group were shown in Table 2, and the clinical characteristics of the preterm birth mothers and term mothers of the validation group were shown in Table 3 and Table 4.

Clinical factors such as gestational age and the like during sampling and delivery were significantly different between the preterm birth group and term birth group, in both the discovery group and validation group (p<0.05, Table 3). Table 3 shows the clinical characteristics of the subjects for validation. The number of white blood cells, and C-reactive protein had a significant difference between two groups in the validation group (p<0.05). Liver function enzymes such as alanine aminotransferase (ALT) and aspartate aminotransferase (AST), and lipid profiles of pregnant women at the third stage of pregnancy had no significant difference between the preterm birth group (preterm birth) and term birth group (term birth) (Table 4). There was no chorioamnionitis in the term birth mothers, but 2 patients were identified in preterm birth women.

**[Table 2]**

| Clinical characteristics of subjects for non-target metabolite profile analysis (discovery group) (n = 21) | | | | |
|---|---|---|---|---|
| | | Term birth (*n* = 10) | Preterm birth (*n* = 11) | p-value |
| | | Median (IQR) | Median (IQR) | |
| Maternal age | | 33.0 (4.8) | 33.0 (4.8) | 0.669* |
| GAS | | 38.5 (1.6) | 31.4 (7.4) | < 0.00*1 |
| Parity | | | | 0.423^{†} |
| | Nulliparous, n | 3 | 4 | |
| | Multiparous, n | 7 | 7 | |
| Gravidity | | | | 0.237^{†} |
| 0, n | | 3 | 7 | |
| ≥ 1, n | | 7 | 4 | |
| WBC ( × 10³/ml) | | 9.8 (2.9) | 10.3 (8.4) | 0.468* |
| C-reactive protein (mg/dl) | | - | 0.5 (0.9) | - |
| AST (IU/L) | | 21.0 (7.3) | 21.0 (3.0) | 0.809* |
| ALT (IU/L) | | 14.5 (3.8) | 14.0 (9.0) | 1.000* |
| AST/ALT ratio | | 1.4 (0.6) | 1.4 (0.7) | 0.918* |
| TC (mg/dl) | | 259.5 (29.0) | 253.0 (36.0) | 0.512* |
| TG (mg/dl) | | 259.0 (115.5) | 178.0 (148.0) | 0.114* |
| BUN (mg/dl) | | 7.0 (1.6) | 6.0 (6.0) | 0.863* |
| Creatinine (mg/dl) | | 0.7 (0.1) | 0.6 (0.2) | 0.223* |
| BUN/Creatinine ratio | | 10.1 (2.9) | 10.1 (8.8) | 0.705* |
| pregBMI | | 25.2 (3.3) | 25.0 (3.7) | 0.029* |
| GAD | | 38.5 (1.6) | 33.0 (8.6) | < 0.001* |
| Delivery mode | | | | 0.063^{†} |
| | Normal delivery, n | 7 | 3 | |
| | Cesarean section, n | 3 | 8 | |
| Birth weight (Kg) | | 3.3 (0.3) | 2.0 (1.5) | < 0.001* |
| Gender, n (%) | | | | 0.426^{†} |
| Male, n | | 7 | 7 | |
| Female, n | | 3 | 4 | |
| APGAR 1min | | 10.0 (0.0) | 6.0 (6.0) | < 0.001* |
| APGAR 5min | | 10.0 (0.0) | 9.0 (3.0) | 0.003* |

| | | | | |
|---|---|---|---|---|
| Data were presented as a median value (interquartile range; IQR). * Mann Whitney test, p < 0.05; ^{†}χ² test, p < 0.05 GAS: gestational age at sampling; PregBMI: body mass index at delivery; GAD: gestational age at delivery. | | | | |

**[Table 3]**

| Clinical characteristics of research subjects of validation group (subjects for validation) | | | | |
|---|---|---|---|---|
| | | Term birth (*n* = 39) | Preterm birth (*n* = 20) | *p*-value |
| Maternal age | | 33.3 ± 0.7 | 32.7 ± 1.0 | 0.626* |
| GAS | | 23.0 ± 0.8 | 27.6 ± 1.6 | 0.010* |
| preBMI | | 21.0 ± 0.4 | 21.7 ± 0.7 | 0.397* |
| Parity | | | | 0.488^{†} |
| | Nulliparous | 16 (42.1) | 8 (40.0) | |
| | Multiparous | 21 (57.9) | 12 (60.0) | |
| Gravidity | | | | 0.416^{†} |
| 0 | | 25 (64.1) | 11 (55.0) | |
| ≥ 1 | | 14 (35.9) | 9 (45.0) | |
| WBC ( × 10³/ml) | | 8.8 ± 0.4 | 11.6 ± 0.6 | <0.001* |
| C-reactive protein (mg/dl) | | 0.3 ± 0.1 | 0.8 ± 0.2 | 0.034* |
| pregBMI | | 26.8 ± 0.6 | 26.4 ± 0.8 | 0.397 |
| GAD | | 39.0 ± 0.2 | 33.3 ± 1.0 | <0.001* |
| Delivery mode | | | | 0.026^{†} |
| | Normal delivery | 23 (59.0) | 5 (25.0) | |
| | Cesarean section | 16 (41.0) | 15 (75.0) | |
| Birth weight (Kg) | | 3.2 ± 0.1 | 22.0 ± 0.2 | <0.001* |
| Gender, n (%) | | | | 0.651^{†} |
| Male | | 25 (64.1) | 14 (70.0) | |
| Female | | 14 (35.9) | 6 (30.0) | |
| APGAR 1min | | 9.7 ± 0.1 | 7.9 ± 0.5 | 0.002 * |
| APGAR 5min | | 9.9 ± 0.1 | 8.9 ± 0.4 | 0.009 * |

| | | | | |
|---|---|---|---|---|
| Data for continuous variables were expressed as mean ± SE. * Student's t-test, p < 0.05; ^{†}χ² test, p < 0.05 TB: term birth; PTB: preterm birth; GAS: gestational age at sampling; PreBMI: body mass index before pregnancy; PregBMI: body mass index at delivery; WBC: white blood cell; GAD: gestational age at delivery; APGAR: Apgar score (Appearance, Pulse, Grimace, Activity, Respiration, Apgar score) | | | | |

**[Table 4]**

| Blood indexes related to retinoid metabolism of term birth and preterm birth mothers (n=59) | | | |
|---|---|---|---|
| Classification | Term Birth (n=39) | Preterm birth (n=20) | *p*-value |
| AST (IU/L) | 16.92 ± 0.70 | 17.75 ± 1.38 | 0.554 |
| ALT (IU/L) | 11.79 ± 1.17 | 12.95 ± 2.00 | 0.597 |
| AST/ALT | 1.69 ± 0.09 | 1.62 ± 0.11 | 0.642 |
| TC (mg/dl) | 263.64 ± 7.07 | 257.75 ± 9.96 | 0.630 |
| TG (mg/dl) | 307.23 ± 24.90 | 264.85 ± 29.62 | 0.303 |
| BUN (mg/dl) | 8.41 ± 0.53 | 7.50 ± 0.77 | 0.328 |
| Creatinine (mg/dl) | 0.57 ± 0.02 | 0.50 ± 0.03 | 0.066 |
| BUN/Creatinine | 14.75 ± 0.56 | 15.60 ± 1.08 | 0.441 |

| | | | |
|---|---|---|---|
| Data were expressed as mean ± SE. * Student's t-test, | | | |

### 2. Metabolite profiling of mother plasma samples

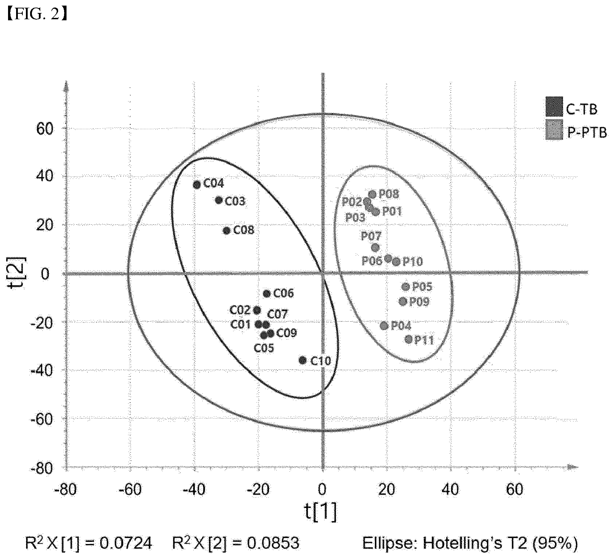

In order to identify metabolites and metabolic pathways specific to preterm birth, non-target metabolite profiling was performed using ultra-high performance liquid chromatography (UPLC) combined with LTQ-Orbitrap Velos Pro hybrid mass spectrometer. Multivariate analysis was performed to obtain a list of variables from the variation in metabolic profiles. Metabolomics patterns which could be used to distinguish the PTB and TB groups were identified by applying score plots of partial least-squares discriminant analysis (PLS-DA). FIG. 2 shows clearly divided categories for PTB compared to TB in RPC positivity (Q2 = 0.072; R2 = 0.085). As shown in FIG. 2, in PLS-DA score plots, the plasma metabolites of the preterm birth mother (red) and term birth mother (blue) were classified differently. Based on the variable importance in projection (VIP) value more than 1 and p-value less than 0.05, 15 metabolites among 235 variable ions were determined. Specific data were shown in Table 5.

**[Table 5]**

| Ranking result of multivariate analysis for variable ions identified by UPLC-LTQ-Orbitrap MS | | | | | | |
|---|---|---|---|---|---|---|
| **No.** | **RT_Mass** | **Detected method** | **VIP value** | **p-value** | **Change** | **Fold change(P/C)** |
| 1 | 11.61_163.0094 | RP | 2.66 | 1.51E-03 | ↑ | 7.41 |
| 2 | 11.36_319.2058 | RP | 2.41 | 3.60E-02 | ↑ | 1.94 |
| 3 | 10.92_274.2816 | RP | 2.09 | 2.63E-02 | ↓ | 0.79 |
| 4 | 5.96_90.5243 | RP | 2.05 | 2.74E-02 | ↑ | 6.51 |
| 5 | 11.63_270.2154 | RP | 1.98 | 1.72E-02 | ↑ | 3.34 |
| 6 | 11.35_133.1127 | RP | 1.85 | 2.50E-02 | ↓ | 0.34 |
| 7 | 11.58_121.102 | RP | 1.54 | 3.07E-02 | ↑ | 8.11 |
| 8 | 10.97_106.0885 | RP | 1.53 | 4.25E-02 | ↓ | 0.73 |
| 9 | 10.2_95.0765 | RP | 1.34 | 4.65E-02 | ↑ | 2.60 |
| 10 | 11.53_834.6856 | RP | 1.30 | 3.95E-02 | ↑ | 61.76 |
| 11 | 2.16_90.5248 | RP | 1.29 | 4.76E-02 | - | - |
| 12 | 11.36_145.0167 | RP | 1.19 | 1.44E-02 | ↑ | 1.75 |
| 13 | 10.99_334.3113 | RP | 1.16 | 3.45E-02 | ↓ | 0.61 |
| 14 | 9.22_95.0809 | RP | 1.00 | 3.87E-02 | ↑ | 2.48 |
| 15 | 11.42_117.0732 | RP | 0.97 | 4.47E-02 | ↑ | 9.68 |
| 16 | 9.67_369.1706 | RN | 1.35 | 2.13E-02 | ↑ | 6.51 |
| 17 | 11.5_685.5345 | RN | 1.35 | 5.63E-03 | ↑ | 11.66 |
| 18 | 0.82_313.0628 | RN | 0.98 | 4.40E-02 | ↑ | 2.20 |

### 3. Metabolic pathway-related preterm birth

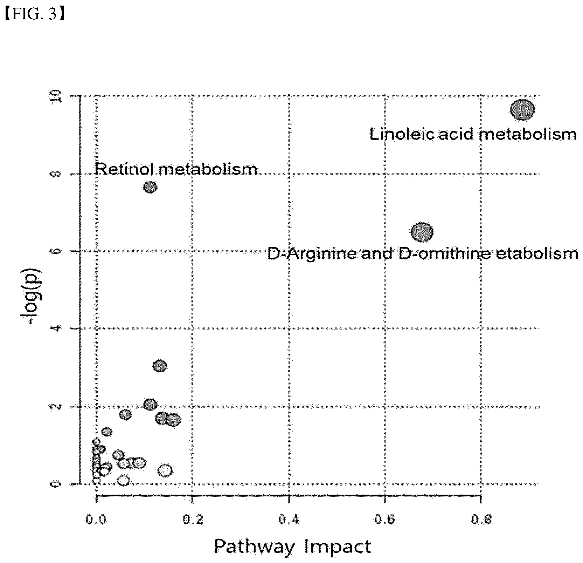

In order to better understand how metabolite changes in pregnant mother plasma are associated with preterm birth, metabolic pathways were analyzed. Pathway analysis (pathway impact analysis) was based on KEGG database and MetaboAnalyst 3.0 for comprehensive metabolic data analysis, visualization and interpretation. The result of the pathway analysis was shown in FIG. 3. FIG. 3 shows a scatter plot of the pathway impact analysis, and the y-axis indicated a p-value. The x-axis indicates pathway topology analysis, and the y-axis indicates pathway enrichment analysis. It was shown that the metabolic pathways including retinol metabolism, linoleic acid metabolism, and D-arginine and D-ornithine metabolism were associated with preterm birth.

### 4. Analysis of target plasma metabolites

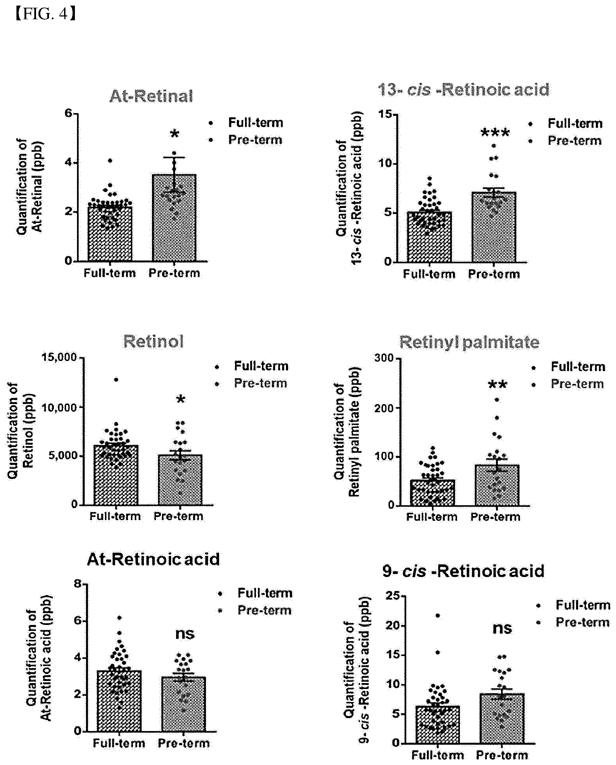

Metabolites involved in retinoid metabolism were analyzed as a target to investigate a pathological mechanism underlying preterm birth. Retinoid metabolites were analyzed in plasma of pregnant women using LC-MS (TB, n = 39; PTB, n = 20). When the gestational age was adjusted, the retinoid metabolites of PTB were significantly changed compared to TB (FIG. 4). FIG. 4 is a drawing which shows retinoid metabolites showing changes in the plasma samples of the preterm birth mothers through retinoid metabolite analysis (39TB, 20PTB). The retinol level was significantly reduced in PTB compared to TB, whereas retinal palmitate, all-trans (AT)-retinal and 13-cis-retinoic acid (13cis-RA) were significantly increased (p<0.05).

### 5. Prediction performance for preterm birth

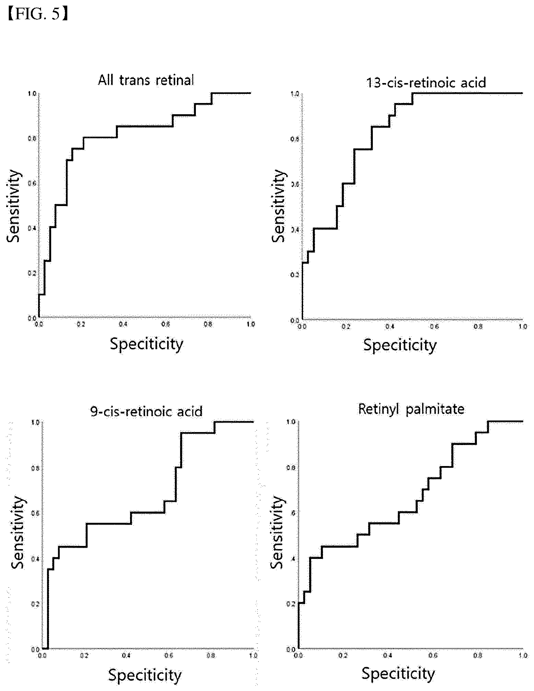

The diagnosis accuracy of preterm birth was verified by applying an AUC (area under the curve) of a ROC (receiver operating characteristic) curve. Retinoid metabolites showed a significant (p <0.01; Table 6, FIG. 5) prediction value of 0.6 or more for preterm birth prediction. FIG. 5 is a drawing which shows ROC curve analysis for metabolites, and shows prediction performance of preterm birth biomarkers using the ROC curve of sensitivity and specificity. AT-retinal (AUC 0.808, 95% CI: 0.683-0.933) and 13cis-RA (AUC 0.826, 95% CI: 0.723-0.930) among the metabolites showed an improved predicting ability. The cut-off value, which is a standard of the metabolite concentration value that distinguishes the preterm birth risk group and normal group, was derived as a metabolite concentration value corresponding to the highest data value among values in which the sensitivity and specificity were added.

**[Table 6]**

| Efficacy of retinol-related metabolites for predicting preterm birth | | | | | | | | |
|---|---|---|---|---|---|---|---|---|
| Metabolite | AUC | p-value | Cut-off value (ppb) | SENS | SPEC | PPV | NPV | Accuracy |
| AT-retinal | 0.808 | < 0.001 | 2.48 | 75.0% | 84.2% | 68.2% | 86.5% | 79.7% |
| 13cis-RA | 0.826 | < 0.001 | 5.56 | 85.0% | 68.4% | 58.6% | 90.0% | 74.6% |
| 9cis-RA | 0.679 | 0.026 | 9.56 | 45.0% | 92.1% | 69.2% | 76.1% | 74.6% |
| Retinyl palmitate | 0.670 | 0.035 | 100.33 | 40.0% | 94.7% | 72.7% | 75.0% | 74.6% |

ROC (Receiver Operating Characteristics) curve analysis was performed for statistical analysis, and p<0.05 was considered significant.
AUC: Area under the curve;
SENS: Sensitivity;
SPEC: Specificity;
PPV: Positive predictive value;
NPV: Negative predictive value;
OR: Odd ratio;
AT: all( 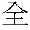 )-trans;
RA: Retinoic acid;
Cut-off value: Critical value

### Conclusion

The present invention confirmed the plasma metabolite profile and metabolic pathway to support the pathogenic mechanism of preterm birth, and investigate a biomarker for predicting preterm birth in pregnant women. According to the present invention, the metabolite profile of the preterm birth group was significantly different from the term birth group, and all-trans-retinal (AT-retinal) and 13-cis-retinoic acid (13cis-RA) among retinol metabolites showed improved prediction of preterm birth.

In the present invention, pregnant mother blood at the second stage or delivery was collected. Despite adjusted gestational age, the plasma metabolite profile of the preterm birth group was different from the term birth group, and the change in retinoid metabolites was associated with preterm birth.

Vitamin A in blood is transported to plasma in a 1:1 complex with a retinol binding protein, RBP. According to the present invention, the plasma retinol level was reduced in PTB compared to TB, but the RBP level related to AT-retinal and 13cis-RA levels was increased in PTB, and these metabolites were positively correlated. RBP can bind to not only retinol but also AT-retinal and 13cis-RA, and can be delivered to target tissue through Stra6 receptor. Specific metabolites of high levels of retinoic acids (ATRA and 13cis-RA) can cause teratogenicity by affecting gene activity during critical organogenesis and embryogenesis.

In summary, the present invention found the metabolite profiles and changes in target metabolites between TB and PTB pregnant mother plasma. In particular, all-trans-retinal (AT-retinal) and 13cis-retinoic acid (13cis-RA) had excellent prediction accuracy for preterm birth.

## Claims

1. A method for providing information for prediction of preterm birth, comprising quantifying at least one metabolite selected from the group consisting of all trans-retinal, 13-cis-retinoic acid, and retinyl palmitate in a biological sample.

2. The method according to claim 1, further comprising comparing the level of the quantified metabolite with a control group.

3. The method according to claim 2, further comprising predicting that there is a risk of preterm birth, when the level of the quantified metabolite is higher than the control group.

4. The method according to claim 3, wherein the control group is a normal birth mother.

5. The method according to claim 1, wherein the biological sample is isolated from a pregnant woman.

6. The method according to claim 1, wherein the biological sample is a blood sample.

7. The method according to claim 1, wherein the biological sample is at least one selected from the group consisting of whole blood, plasma and serum.

8. The method according to claim 1, wherein the quantifying the metabolite is performed by at least one selected from the group consisting of liquid chromatography-mass spectrometer, nuclear magnetic resonance spectrometer, ultraviolet spectrometer, infrared spectrometer, fluorescence spectrometer, ELISA (enzyme-linked immunosorbent assay), and mass spectrometer.

9. The method according to claim 1, wherein the preterm birth is preterm birth that is not caused by liver dysfunction.

10. The method according to claim 1, wherein the biological sample is isolated from a mother having no liver dysfunction.

11. A composition for predicting preterm birth, comprising an agent capable of quantifying at least one metabolite selected from the group consisting of all trans-retinal, 13-cis-retinoic acid, and retinyl palmitate in a biological sample.

12. The composition according to claim 11, wherein the agent can quantify an increase in the level of the metabolite.

13. The composition according to claim 11, wherein the biological sample is isolated from a pregnant woman.

14. The composition according to claim 11, wherein the biological sample is a blood sample.

15. The composition according to claim 11, wherein the biological sample is at least one selected from the group consisting of whole blood, plasma and serum.

16. The composition according to claim 11, wherein the metabolite is quantified with at least one selected from the group consisting of liquid chromatography-mass spectrometer, nuclear magnetic resonance spectrometer, ultraviolet spectrometer, infrared spectrometer, fluorescence spectrometer, ELISA (enzyme-linked immunosorbent assay), and mass spectrometer.

17. A kit for predicting preterm birth, comprising an agent for quantification, or a device for quantification of at least one metabolite selected from the group consisting of all trans-retinal, 13-cis-retinoic acid, and retinyl palmitate in a biological sample.

18. The kit according to claim 17, wherein the kit quantifies an increase in the level of the metabolite.

19. The kit according to claim 17, wherein the biological sample is isolated from a pregnant woman.

20. The kit according to claim 17, wherein the biological sample is a blood sample.

21. The kit according to claim 17, wherein the biological sample is at least one selected from the group consisting of whole blood, plasma and serum.

22. The kit according to claim 17, wherein the device for quantification is at least one selected from the group consisting of liquid chromatography-mass spectrometer, nuclear magnetic resonance spectrometer, ultraviolet spectrometer, infrared spectrometer, fluorescence spectrometer, ELISA (enzyme-linked immunosorbent assay), and mass spectrometer.

23. A system for predicting preterm birth, comprising an input unit which receives input of a biological sample of a test subject;
a quantification unit which quantifies at least one metabolite selected from the group consisting of all trans-retinal, 13-cis-retinoic acid, and retinyl palmitate in the sample;
a calculation unit which compares the level of the quantified metabolite with a control group; and
a display unit which displays a result indicating whether there is a risk of preterm birth of the test subject according to the comparison result.

24. The system according to claim 23, wherein the display unit displays that the test subject has a risk of preterm birth, when it is calculated that the level of the metabolite is higher than the control group in the calculation unit.

25. The system according to claim 23, wherein the biological sample is isolated from a pregnant woman.

26. The system according to claim 23, wherein the biological sample is a blood sample.

27. The system according to claim 23, wherein the biological sample is at least one selected from the group consisting of whole blood, plasma and serum.

28. The system according to claim 23, wherein the quantification unit quantifies the metabolite with at least one selected from the group consisting of liquid chromatography-mass spectrometer, nuclear magnetic resonance spectrometer, ultraviolet spectrometer, infrared spectrometer, fluorescence spectrometer, ELISA (enzyme-linked immunosorbent assay), and mass spectrometer.
